# EUROPÄISCHE PATENTSCHRIFT

(11) **EP 3 344 329 B1**
(45) Veröffentlichungstag und Bekanntmachung des Hinweises auf die Patenterteilung: **04.09.2019**
(21) Anmeldenummer: 16754175.4
(22) Anmeldetag: 27.07.2016
(51) Int. Cl.: A61N 1/05, A61N 1/36

(54) **COCHLEA-IMPLANTAT**
COCHLEAR IMPLANT
IMPLANT COCHLÉAIRE

(30) Priorität: 31.08.2015 DE 102015114514
(43) Veröffentlichungstag der Anmeldung: 11.07.2018
(73) Patentinhaber: Medizinische Hochschule Hannover, 30625 Hannover (DE); Gottfried Wilhelm Leibniz Universität Hannover, 30167 Hannover (DE)
(72) Erfinder: BLUME, Holger, 30900 Wedemark (DE); ERTMER, Wolfgang, 30823 Garbsen (DE); LENARZ, Thomas, 30559 Hannover (DE); KRAL, Andrej, 30175 Hannover (DE)
(74) Vertreter: Patentanwälte Thömen & Körner
(86) Internationale Anmeldenummer: PCT/DE2016/100342
(87) Internationale Veröffentlichungsnummer: WO 2017/036441

(56) Entgegenhaltungen:
- US-A- 5 061 282
- US-A1- 2002 029 070
- US-A1- 2013 148 828

## Beschreibung

Die Erfindung betrifft ein Cochlea-Implantat nach dem Oberbegriff des Anspruchs 1.

Heute verfügbare Cochlea-Implantate sind in ihrer Leistungsfähigkeit durch die Anzahl an Elektroden, die auf ihnen implementiert werden können, limitiert. Jede Elektrode wird durch einen eigenen Draht, der im Elektrodenträger einzeln geführt werden muss, einerseits mit Energie versorgt und andererseits auch elektronisch angesteuert. Die bestromten Elektroden stimulieren Fasern des Hörnervs. Die Verteilung der Rezeptoren, die mit Hörnervfasern verbunden sind, verläuft für die unterschiedlichen Schall-Frequenzen in der Cochlea so, dass die hohen Frequenzen mehr zur Schneckenbasis und die tiefen Frequenzen mehr zur Schneckenspitze, dem Helicotrema, abgebildet werden. Diese Organisation wird in der elektrischen Stimulation genutzt (Ortscode).

Typische kommerzielle Systeme kommen heute auf eine Anzahl von 23 Elektroden. Eine weitere Zunahme dieser Elektroden-Anzahl ist limitiert durch die Steifigkeit des Elektrodenträgers (Cochlea-Implantats). Mit jeder weiteren Elektrode führen die zusätzlichen Drähte zu einer weiteren Versteifung des Elektrodenträgers, was eine Einbringung in die menschliche Cochlea erschwert und die Wahrscheinlichkeit der Verletzung des Innenohrs steigert. Andererseits reicht die geringe Zahl von 23 Elektroden nicht aus, um die für eine gute Hörqualität erforderliche Frequenzauflösung zu erzielen.

Aus der DE 100 18 361 A1 ist ein Cochlea-Implantat eines Cochlea-Implantat-Systems bekannt. Neben der Erwähnung einer dem Stand der Technik gemäß dem Oberbegriff des Patentanspruchs 1 zuzurechnenden Ausführung mit Elektroden, die mechanisch miteinander verbunden und in Form einer Schnecke aneinander gereiht in eine Cochlea zur Stimulation von Rezeptoren des Hörnervs einsetzbar sind, wird in der Druckschrift als Gegenstand, für den Schutz begehrt wird, eine Ausführung mit elektromechanischen Wandlern beschrieben. Diese Wandler werden ebenfalls in Form einer Schnecke aneinander gereiht in eine Cochlea eingesetzt, regen jedoch die flüssigkeitsgefüllten Innenohrräume des geschädigten Innenohrs durch eine dynamische Volumenänderung an. Dabei werden die Wandler derart elektrisch ansteuert, dass auf der Basilarmembran des geschädigten Innenohres eine Wanderwellenkonfiguration entsteht, die die Art der Wander wellenausbildung eines gesunden, nicht geschädigten Innenohres approximiert.

Als Weiterbildung kann der in der vorgenannten Druckschrift beschriebene Gegenstand eine Dekodierlogik und Umsetzerbausteine enthalten, die den Anschluss einer polreduzierten Implantatleitung ermöglichen. So kann beispielhaft der Arrayanschluss aus nur drei Leitungen für Masse, Daten und ein Taktsignal bestehen, wobei die notwendige Versorgung mit elektrischer Betriebsenergie durch Phantomspeisung auf der Taktsignalleitung erfolgen kann.

Der Erfindung liegt die Aufgabe zugrunde, ein Cochlea-Implantat zu schaffen, das mehr als die bisher übliche Anzahl von Elektroden unter Beibehaltung oder Verringerung seiner Steifigkeit umfasst. Zusätzlich soll die benötigte implantierte Elektronik vereinfacht werden.

Diese Aufgabe wird bei einem Cochlea-Implantat nach dem Oberbegriff des Anspruchs 1 durch die Merkmale dieses Anspruchs gelöst.

Weiterbildungen und vorteilhafte Ausgestaltungen der Erfindung ergeben sich aus den Unteransprüchen.

Bei der Erfindung werden die Elektroden nicht mehr zentral über gesonderte eigene Drähte bestromt, sondern als Bestandteile von Modulen mit eigenen Dekodierungs- und Steuerschaltungen autonom bestromt, indem empfangene codierte Moduladressen und Stimulationssignale decodiert, ausgewertet und ausgeführt werden. Es wird so die Möglichkeit geschaffen, die Anzahl der die Module mit elektrischer Energie versorgenden Drähte zu verringern. Bei einer galvanischen Einspeisung sind zwei Drähte ausreichend, bei einer induktiven oder kapazitiven Einspeisung können sogar Drähte zwischen den einzelnen Modulen entfallen. Dadurch wird die Steifigkeit des Cochlea-Implantats nicht mehr von der Anzahl der zuführenden Elektrodendrähte beeinflusst und gegenüber den bisherigen Systemen mit 23 Elektroden deutlich verringert.

Moduladressen enthalten Angaben darüber, welches Modul oder welche Module angesprochen werden und damit auch, welche Elektroden bestromt werden. Damit werden gleichzeitig das Teilfrequenzband oder die Teilfrequenzbänder des genutzten Hörspektrums ausgewählt.

Stimulationssignale enthalten Angaben darüber, mit welcher Impulsfrequenz die Elektroden bestromt werden. Die Impulsfrequenz ist ein Maß für die empfundene Tonhöhe. Darüber hinaus können die Stimulationssignale optional Angaben über die Impulsamplitude, das Puls-Pausen-Verhältnis und die Pulsform enthalten.

Bei einer ersten Variante können die Module galvanisch über eine gemeinsame Zweidrahtleitung mit der Energiequelle verbunden sein.

In diesem Fall kann die Energiequelle abgesetzt von den einzelnen Modulen angeordnet und für die Energiespeicherung oder die Aufnahme drahtlos empfangener Energie optimiert sein.

Bei einer zweiten Variante können die Module induktiv oder kapazitiv mit einer ein elektromagnetisches Wechselfeld erzeugenden Energiequelle gekoppelt sein, wobei jedes Modul oder jede Gruppe von wenigstens zwei galvanisch untereinander verbundenen Modulen eine induktive oder kapazitive Energieempfangsantenne und eine Gleichrichterschaltung umfasst.

Bei dieser Variante werden jedes Modul oder zu einer Gruppe zusammengefasste Module gesondert mit Energie versorgt. Eine gemeinsame galvanische Verbindung aller Module untereinander ist nicht erforderlich. Die Steifigkeit des Cochlea-Implantats kann dadurch noch weiter verringert werden. Außerdem beschränkt sich das Cochlea-Implantat auf die aneinandergereihten Module und benötigt keine weiteren zu implantieren Komponenten, was die gesamte implantierte Komponente deutlich verkleinert.

Auch bei der Datenübertragung zu den einzelnen Modulen sind unterschiedliche Varianten möglich. So können die codierten Moduladressen und Stimulationssignale den Eingängen der Decoder- und Steuerschaltung über einen Datenbus zugeführt werden. Diese Lösung kommt mit einem gemeinsamen Empfänger für die an die Module zu übermittelnden codierten Moduladressen und Stimulationssignale aus. Die Moduladressen und Stimulationssignale werden über den Datenbus allen Modulen zugeführt und die Module entscheiden anhand der Moduladressen, ob sie Elektroden bestromen sollen oder nicht.

Der Datenbus kann aus der gemeinsamen Zweidrahtleitung bestehen.

Dadurch ist es möglich, dieselbe Leitung sowohl zur Energieübertragung als auch zur Datenübertragung zu nutzen.

Bei einer anderen Variante können die codierten Moduladressen und Stimulationssignale den Eingängen der Decoder- und Steuerschaltung jeweils über einen eigenen Empfänger mit einer Signalempfangsantenne zugeführt werden, wobei der Empfänger mit der Signalempfangsantenne ein von einem Sender übertragenes elektromagnetisches Signal empfängt, das mit den codierten Moduladressen und Stimulationssignalen moduliert ist.

Analog zu den Vorteilen einer für jedes Modul gesonderten Energieversorgung ist eine gemeinsame galvanische Verbindung aller Module untereinander nicht erforderlich. Die Steifigkeit des Cochlea-Implantats kann dadurch noch weiter verringert werden. Außerdem beschränkt sich das Cochlea-Implantat auf die aneinandergereihten Module und benötigt keine weiteren zu implantieren Komponenten.

Das mit codierten Moduladressen und Stimulationssignalen modulierte elektromagnetische Signal kann die gleiche Trägerfrequenz wie das energieübertragende elektromagnetische Wechselfeld haben.

In diesem Fall müssen der Sender und Empfänger sowie die Sende- und Empfangsantenne nur jeweils einfach vorhanden sein. Der Aufwand für die einzusetzenden Komponenten wird so verringert. Allerdings stellt diese Lösung einen Kompromiss zwischen der Eindringtiefe des elektromagnetischen Wechselfeldes und der maximal übertragbaren Datenrate dar.

Dies ist nicht der Fall, wenn gemäß einer Ausführungsform das mit codierten Moduladressen und Stimulationssignalen modulierte elektromagnetische Signal eine höhere Trägerfrequenz als das energieübertragende elektromagnetische Wechselfeld hat.

In dem Fall kann mit einer niedrigen Trägerfrequenz Energie über ein Wechselfeld übertragen werden, ohne dass durch das Körpergewebe eine zur Erwärmung führende Dämpfung auftritt. Mit einer hohen Trägerfrequenz können die codierten Moduladressen und Stimulationssignale übertragen werden, wenn für eine präzise selektive Steuerung der Module eine hohe Datenrate benötigt wird. Die höhere Dämpfung gegenüber niedrigen Frequenzen kann in Kauf genommen werden, da keine Versorgungsenergie übertragen werden muss, sondern nur Signale mit einer Leistung, die für eine Lesbarkeit und Auswertung ausreicht. Der Vorteil hoher Trägerfrequenzen besteht auch darin, dass eine starke Bündelung durch eine Antenne mit einem hohen Antennengewinn möglich ist und dadurch die Hochfrequenzleistung reduziert werden kann. Außerdem nähert sich die Wellenlänge den maximal möglichen Abmessungen der in den Modulen vorhandenen Antennen an. Denn diese Abmessungen sind durch die Cochlea vorgegeben.

Nachfolgend wird die Erfindung anhand eines Ausführungsbeispiels erläutert, dass in der Zeichnung dargestellt ist. Darin zeigen:
- Fig. 1: ein Ausführungsbeispiel des Cochlea-Implantat-Systems,
- Fig. 2: Einzelheiten eines Cochlea-Implantats mit Datenbus
- Fig. 3: Einzelheiten eines Cochlea-Implantats mit Signal - und Energieübertragung auf der gleichen Frequenz für jedes Modul gesondert und
- Fig. 4: Einzelheiten eines Cochlea-Implantats mit Signal- und Energieübertragung auf unterschiedlichen Frequenzen.

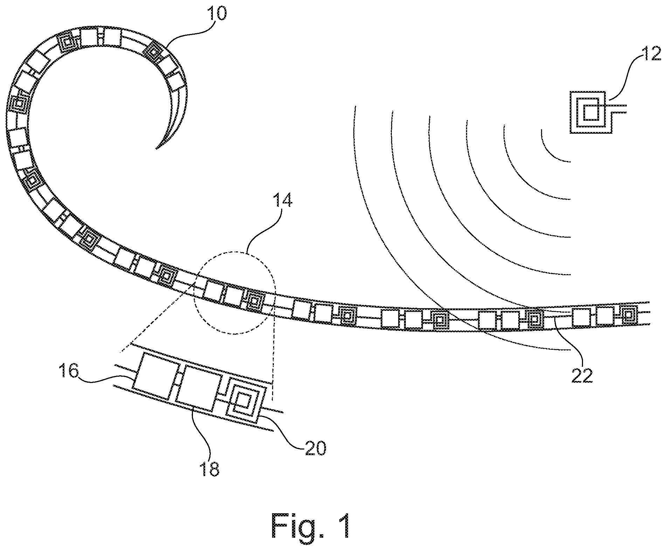

Fig. 1 zeigt ein Ausführungsbeispiel eines Cochlea-Implantat-Systems. Das Cochlea Hörsystem besteht aus einem externen Teil, welches ein Mikrofon, einen Sprachprozessor, eine Energiequelle und einen Sender mit einer Sendeantenne umfasst sowie aus einem implantierten Teil. Der externe Teil (der Sprachprozessor) wird in bisher bekannter Weise meist hinter dem Ohr getragen. In Fig. 1 ist der externe Teil lediglich als Spulensymbol 12 vereinfacht dargestellt. Der implantierte Teil des Cochlea-Implantat-Systems besteht aus dem schneckenförmigen Cochlea-Implantat 10 sowie einem hier nicht dargestellten Energieempfänger mit einer Empfangsantenne und einem Gleichrichter.

Das Cochlea-Implantat 10 umfasst Module 14, die ihrerseits jeweils eine Elektrode 16, eine Decoder-und Steuerschaltung 18 sowie einen Signalempfänger 20 mit einer Empfangsantenne beinhalten. Die Module 14 sind mit der Energiequelle, in diesem Fall dem implantierten Gleichrichter, über eine gemeinsame Zweidrahtleitung galvanisch verbunden und gemeinsam gekapselt.

Die Elektroden 16 der Module 14 sind den Fasern des Hörnervs benachbart angeordnet und werden zur Stimulierung der Fasern impulsartig bestromt. Jedes Modul 14 repräsentiert einen Teilfrequenzbereich des genutzten Hörspektrums. Je höher die Anzahl und räumliche Dichte der Module 14 realisiert werden kann, desto besser ist die Auflösung des genutzten Hörspektrums in Teilfrequenzbereiche oder die Erweiterung des genutzten Hörspektrums, was zu einer Verbesserung der Hörqualität führt. Das Lautstärkeempfinden wird durch die Amplitude und die Gesamtzahl der Stromimpulse, die die Rezeptoren stimulieren, innerhalb eines Zeitraums bestimmt. Je nach Komplexität des ursprünglichen akustischen Signals können eine Elektrode 16 oder mehrere Elektroden gemeinsam die Hörnervfasern stimulieren. Bei mehreren Elektroden ist wiederum eine sequenzielle oder quasi parallele Bestromung möglich. Unter quasi parallel wird eine durch die serielle Datenübertragung und Auswertung hervorgerufene geringfügige zeitliche Verschiebung der Steuerbefehle und damit der zeitlichen Anfänge der Bestromungen der Elektroden verstanden, die physiologisch allerdings unbeachtlich ist. Auch in der physiologischen Bedingung des normalhörenden Ohrs kommt es zu einem zeitlichen Versatz der Erregung entlang der Cochlea ("Wanderwelle").

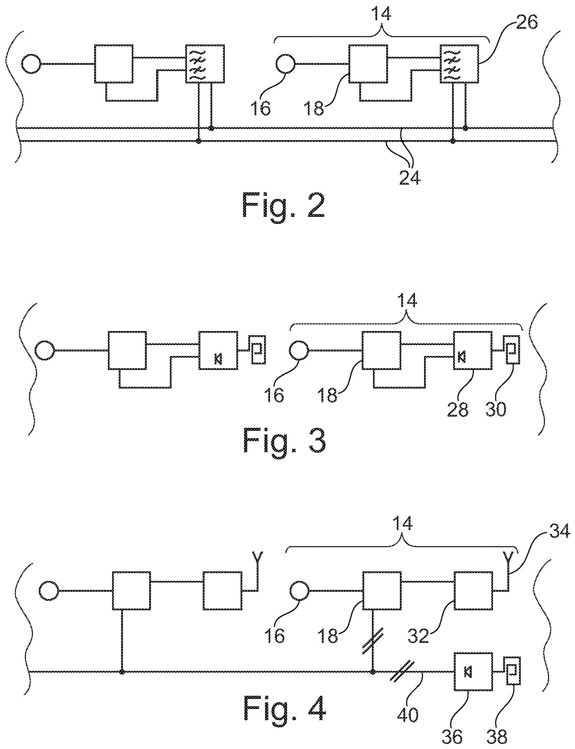

Fig. 2 zeigt Einzelheiten eines Cochlea-Implantats 10 mit Datenbus. Ein durchgehender Datenbus 24, der auch zur Energieversorgung der einzelnen Module 14 verwendet werden kann, führt zu allen Eingängen der Decoder- und Steuerschaltungen 18. Nach Dekodierung der Moduladressen wird entschieden, ob das jeweilige Modul angesprochen ist oder nicht. Falls es angesprochen ist, wird das Stimulationssignal ausgewertet und in Stromimpulse umgesetzt, die über die Elektroden 16 die Rezeptoren stimulieren. Das Stimulationssignal beinhaltet Angaben über die Pulsfrequenz sowie optional über das Puls-Paulsen-Verhältnis, die Anzahl der Pulse, die Pulsamplitude sowie die Pulsform. Falls die Drähte des Datenbusses 24 auch zur Energieversorgung verwendet werden, werden die codierten Signale mit den Moduladressen und Stimulationssignalen über eine Weiche 26 getrennt.

Fig. 3 zeigt Einzelheiten eines Cochlea-Implantats 10 mit Signal- und Energieübertragung auf der gleichen Frequenz für jedes Modul 14 gesondert. In jedem Modul 14 ist vor der Decoder- und Steuerschaltung 18 ein Empfänger 28 mit einer Empfangsantenne 30 angeordnet. Ein Signalausgang des Empfängers 28 ist mit einem Eingang der Decoder- und Steuerschaltung 18 verbunden. Ein Energieausgang des Empfängers 28 ist mit einem Gleichrichter verbunden, dessen Ausgang wiederum mit einem Energieversorgungseingang der Decoder- und Steuerschaltung 18 verbunden ist.

Figur 4 zeigt Einzelheiten eines Cochlea-Implantats 10 mit Signal- und Energieübertragung auf unterschiedlichen Frequenzen. In jedem Modul 14 ist vor der Decoder- und Steuerschaltung 18 ein Empfänger 32 mit einer Empfangsantenne 34 angeordnet. Der Empfänger 32 mit der Empfangsantenne 34 ist für eine Signalübertragung auf einer hohen Trägerfrequenz bemessen. Ein Signalausgang des Empfängers 32 ist mit einem Eingang der Decoder- und Steuerschaltung 18 verbunden.

Für die Energieversorgung ist ein für alle Module 14 gemeinsamer Energieempfänger 36 mit Empfangsantenne 38 für niedrige Frequenzen vorhanden. Ein Energieausgang des Empfängers 36 ist mit einem Gleichrichter verbunden dessen Ausgang wiederum mit einer zu allen Modulen 18 führenden durchgeschleiften Zweidrahtleitung 40 verbunden ist.

## Patentansprüche

1. Cochlea-Implantat (10) eines Cochlea-Implantat-Systems, bestehend aus Elektroden (16), die mechanisch miteinander verbunden und in Form einer Schnecke aneinandergereiht in eine Cochlea zur Stimulation von Rezeptoren des Hörnervs einsetzbar sind, sowie aus einer die einzelnen Elektroden (16) gezielt mit elektrischen Impulsen beaufschlagbaren Steuerschaltung (18) und einer Energiequelle, wobei mit der Steuerschaltung (18) die Elektroden (16) mit aus den akustischen Signalen durch einen Prozessor erzeugten elektrischen Signalen impulsartig bestromt und den Elektroden (16) benachbarte Rezeptoren des Hörnervs stimuliert werden, **dadurch gekennzeichnet, dass** die Elektroden (16) Bestandteile von Modulen (14) sind, die jeweils eine eigene Decoder- und Steuerschaltung (18) zur Decodierung von Moduladressen und Stimulationssignalen sowie zur Erzeugung elektrischer Impulse für die Elektroden (16) umfassen, wobei aus akustischen Signalen erzeugte codierte Moduladressen und Stimulationssignale an Eingängen der Decoder- und Steuerschaltung (18) anliegen.

2. Cochlea-Implantat nach Anspruch 1, **dadurch gekennzeichnet, dass** die Module (14) galvanisch über eine gemeinsame Zweidrahtleitung (22; 24) mit der Energiequelle verbunden sind.

3. Cochlea-Implantat nach Anspruch 1, **dadurch gekennzeichnet, dass** die Module (14) induktiv oder kapazitiv mit einer ein elektromagnetisches Wechselfeld erzeugenden Energiequelle gekoppelt sind, wobei jedes Modul (14) oder jede Gruppe von wenigstens zwei galvanisch untereinander verbundenen Modulen eine induktive oder kapazitive Energieempfangsantenne (20; 30; 38) und eine Gleichrichterschaltung (28; 36) umfasst.

4. Cochlea-Implantat nach einem der Ansprüche 1 - 3, **dadurch gekennzeichnet, dass** die codierten Moduladressen und Stimulationssignale den Eingängen der Decoder- und Steuerschaltung (18) über einen Datenbus (40) zugeführt sind.

5. Cochlea-Implantat nach Anspruch 4, **dadurch gekennzeichnet, dass** der Datenbus (40) aus der gemeinsamen Zweidrahtleitung besteht.

6. Cochlea-Implantat nach einem der Ansprüche 1 - 3, **dadurch gekennzeichnet, dass** die codierten Moduladressen und Stimulationssignale den Eingängen der Decoder- und Steuerschaltung (18) jeweils über einen eigenen Empfänger mit einer Signalempfangsantenne ((26) zugeführt sind, wobei der Empfänger mit der Signalempfangsantenne ein von einem Sender (12) übertragenes elektromagnetisches Signal empfängt, das mit den codierten Moduladressen und Stimulationssignalen moduliert ist.

7. Cochlea-Implantat nach Anspruch 6, **dadurch gekennzeichnet, dass** das mit codierten Moduladressen und Stimulationssignalen modulierte elektromagnetische Signal die gleiche Trägerfrequenz wie das energieübertragende elektromagnetische Wechselfeld hat.

8. Cochlea-Implantat nach Anspruch 6, **dadurch gekennzeichnet, dass** das mit codierten Moduladressen und Stimulationssignalen modulierte elektromagnetische Signal eine höhere Trägerfrequenz als das energieübertragende elektromagnetische Wechselfeld hat.

## Claims

1. Cochlear implant (10) of a cochlear implant system, consisting of electrodes (16) that are mechanically connected to one another and usable, strung together in the form of a spiral, in a cochlea for stimulating receptors of the cochlear nerve, and of a control circuit (18) that can apply electrical pulses to the individual electrodes (16) in targeted fashion and an energy source, wherein, by way of the control circuit (18), the electrodes (16) are energized in pulse-like fashion by electrical signals that are produced by a processor from the acoustic signals and receptors of the cochlear nerve adjacent to the electrodes (16) are stimulated, **characterized in that** the electrodes (16) are constituent parts of modules (14), which each comprise a dedicated decoding and control circuit (18) for decoding module addresses and stimulation signals and for producing electrical pulses for the electrodes (16), wherein encoded module addresses and stimulation signals produced from acoustic signals are present at the inputs of the decoding and control circuit (18).

2. Cochlear implant according to Claim 1, **characterized in that** the modules (14) are electrically connected to the energy source by way of a common two-wire cable (22; 24).

3. Cochlear implant according to Claim 1, **characterized in that** the modules (14) are inductively or capacitively coupled to an energy source that produces an alternating electromagnetic field, wherein each module (14) or each group of at least two modules electrically connected to one another comprises an inductive or capacitive energy reception antenna (20; 30; 38) and a rectifier circuit (28; 36).

4. Cochlear implant according to any one of Claims 1-3, **characterized in that** encoded module addresses and stimulation signals are supplied to the inputs of the decoding and control circuit (18) by way of a data bus (40).

5. Cochlear implant according to Claim 4, **characterized in that** the data bus (40) consists of the common two-wire cable.

6. Cochlear implant according to any one of Claims 1-3, **characterized in that** the encoded module addresses and stimulation signals are supplied in each case to the decoding and control circuit (18) by way of a dedicated receiver with a signal reception antenna (26), wherein the receiver receives by means of the signal reception antenna an electromagnetic signal transmitted by a transmitter (12), said electromagnetic signal being modulated by the encoded module addresses and stimulation signals.

7. Cochlear implant according to Claim 6, **characterized in that** the electromagnetic signal modulated by encoded module addresses and stimulation signals has the same carrier frequency as the energy-transmitting alternating electromagnetic field.

8. Cochlear implant according to Claim 6, **characterized in that** the electromagnetic signal modulated by encoded module addresses and stimulation signals has a higher carrier frequency than the energy-transmitting alternating electromagnetic field.

## Revendications

1. Implant cochléaire (10) d'un système d'implant cochléaire, constitué d'électrodes (16), qui sont reliées mécaniquement entre elles et qui peuvent être insérées en série sous la forme d'une vis dans une cochlée pour stimuler des récepteurs du nerf auditif, ainsi que d'un circuit de commande (18) qui peut solliciter les électrodes individuelles (16) de manière ciblée avec des impulsions électriques, et d'une source d'énergie, dans lequel le circuit de commande (18) permet d'exciter de manière impulsionnelle les électrodes (16) avec des signaux électriques générés par un processeur à partir des signaux acoustiques, et de stimuler des récepteurs du nerf auditif adjacent aux électrodes (16), **caractérisé en ce que** les électrodes (16) sont des composants de modules (14) qui comprennent chacun un circuit de décodage et de commande propre (18) destiné à décoder des adresses de modules et des signaux de stimulation ainsi qu'à générer des impulsions électriques destinées aux électrodes (16), dans lequel des adresses de modules codées et des signaux de stimulation codés, générés à partir de signaux acoustiques, sont appliqués à des entrées du circuit de décodage et de commande (18).

2. Implant cochléaire selon la revendication 1, **caractérisé en ce que** les modules (14) sont reliés de manière galvanique à la source d'énergie par l'intermédiaire d'une ligne bifilaire commune (22 ; 24).

3. Implant cochléaire selon la revendication 1, **caractérisé en ce que** les modules (14) sont couplés de manière inductive ou capacitive à une source d'énergie générant un champ électromagnétique alternatif, dans lequel chaque module (14) ou chaque groupe d'au moins deux modules reliés entre eux de manière galvanique, comprend une antenne de réception d'énergie inductive ou capacitive (20 ; 30 ; 38) et un circuit redresseur (28 ; 36).

4. Implant cochléaire selon l'une des revendications 1 à 3, **caractérisé en ce que** les adresses de modules codées et les signaux de stimulation codés sont appliqués aux entrées du circuit de décodage et de commande (18) par l'intermédiaire d'un bus de données (40).

5. Implant cochléaire selon la revendication 4, **caractérisé en ce que** le bus de données (40) est constitué de la ligne bifilaire commune.

6. Implant cochléaire selon l'une des revendications 1 à 3, **caractérisé en ce que** les adresses de modules codées et les signaux de stimulation codés sont respectivement appliqués aux entrées du circuit de décodage et de commande (18) par l'intermédiaire d'un récepteur distinct comportant une antenne de réception de signal (26), dans lequel le récepteur reçoit au moyen de l'antenne de réception de signal un signal électromagnétique transmis par un émetteur (12), lequel signal est modulé avec les adresses de modules codées et les signaux de stimulation codés.

7. Implant cochléaire selon la revendication 6, **caractérisé en ce que** le signal électromagnétique modulé avec des adresses de modules codées et des signaux de stimulation codés présente la même fréquence porteuse que le champ électromagnétique alternatif transmettant de l'énergie.

8. Implant cochléaire selon la revendication 6, **caractérisé en ce que** le signal électromagnétique modulé avec des adresses de modules codées et des signaux de stimulation codés présente une fréquence porteuse plus élevée que le champ électromagnétique alternatif transmettant de l'énergie.
